# DEMANDE DE BREVET EUROPEEN

(11) **EP 2 733 631 A1**
(43) Date de publication de la demande: **21.05.2014**
(21) Numéro de dépôt: 12306421.4
(22) Date de dépôt: 15.11.2012
(51) Int. Cl.: G06F 19/00

(54) **Système pour la tracabilité automatique des dispositifs médicaux**

(71) Demandeur: Visiodent, 93210 La Plaine Saint Denis (FR)
(72) Inventeur: Sebag, Jacques, 78170 La Celle Saint -Cloud (FR); Ohnona, Michel, 75017 Paris (FR)
(74) Mandataire: Hirsch & Associés

(57) **Abrégé**

Système (S) pour la traçabilité des dispositifs médicaux utilisés pour le soin de patients, comportant une interface physique (M1) pour déterminer automatiquement un identifiant (I_{M}) de dispositif médical courant (M), une interface homme-machine (M2) pour déterminer un identifiant (Iₚ) de patient courant (P), une base de données (DB) associant des identifiants de patients avec un ensemble d'identifiants de dispositifs médicaux utilisés, et des moyens de traitement (MT) pour insérer cet identifiant (I_{M}) de dispositif médical courant (M) dans la base de données (DB) en association avec l'identifiant (Ip) de patient courant (P).

## Description

La présente invention concerne la traçabilité automatique des dispositifs médicaux utilisés dans un cabinet médical.

Il est important pour les structures de soin (services hospitaliers, cliniques, cabinets médicaux etc.) de pouvoir tracer les dispositifs médicaux utilisés lors des différentes interventions.

Un dispositif médical peut être défini comme tout instrument, appareil, équipement, matière ou autre article, utilisé seul ou en association, destiné à être utilisé chez un patient à des fins de diagnostic, de prévention, de traitement ou d'atténuation d'une maladie, d'une blessure, d'un handicap, etc., d'étude, de traitement ou de modification de l'anatomie, de maîtrise de la conception... Un dispositif médical peut comprendre également des moyens logiciels mais on exclue généralement de sa définition les moyens pharmacologiques ou immunologiques.

Des définitions sont fournies par l'article L.5211-1 du Code de la santé publique ou par la directive européenne 93/42/CE. D'autres définitions peuvent toutefois s'appliquer en fonction, notamment, des législations nationales.

Cette traçabilité permet aux praticiens (médecins, infirmiers, personnels soignants en général) d'être en mesure de répondre aux patients ou à l'administration sur les produits utilisés, et ce plusieurs mois ou années après l'intervention.

Elle répond en outre de plus en plus à des exigences légales. Ainsi, en France, l'article L.5212-3 du Code de la Santé Publique stipule les modalités de mise en oeuvre de cette traçabilité des produits médicaux au sein des structures de soin. Il leur impose notamment de conserver en liaison aux dossiers des patients toutes les informations sur les produits médicaux utilisés lors de chaque séance de soin.

Dans le cadre particulier d'un cabinet ou d'une clinique dentaire, cela comprend les informations sur les matériaux, dispositifs implantables, cycles de stérilisation des instruments, prothèses dentaires et consommables en général.

Cette exigence légale vise notamment un objectif de sécurité sanitaire. Ainsi, en cas de rappel d'un produit décidé par le fabriquant ou le distributeur du produit, ou bien par l'administration, le service de soin doit être en mesure de déterminer l'ensemble des patients qui ont été traités avec le produit en question.

Il est toutefois en pratique très difficile de répondre à une telle exigence pour un service de soins, notamment du fait du très grand nombre de dispositifs médicaux différents susceptibles d'être utilisés. Cette exigence est particulièrement contraignante dans le contexte d'un cabinet médical ou dentaire dans lequel le praticien doit gérer seul la gestion des dispositifs médicaux utilisés et la noter dans les dossiers personnels de chaque patient.

De fait, l'exigence légale est peu respectée et il en résulte une lacune importante en matière de matériovigilance.

Le but de la présente invention est de fournir un procédé et un système palliant au moins partiellement les inconvénients précités. Plus particulièrement, l'invention vise notamment à automatiser le traçage des dispositifs médicaux et leur association avec les dossiers des patients.

À cette fin, la présente invention propose un système pour la traçabilité des dispositifs médicaux utilisés pour le soin de patients, comportant
- une interface physique pour déterminer automatiquement un identifiant de dispositif médical courant,
- une interface homme-machine pour déterminer un identifiant de patient courant,
- une base de données associant des identifiants de patients avec un ensemble d'identifiants de dispositifs médicaux utilisés, et
- des moyens de traitement pour insérer ledit identifiant de dispositif médical courant dans ladite base de données en association avec ledit identifiant de patient courant.

Suivant des modes de réalisation préférés, l'invention comprend une ou plusieurs des caractéristiques suivantes :
- ladite interface physique comporte un moyen de lecture d'informations visuelles apposées sur ledit dispositif médical courant.
- ladite interface physique comporte un moyen de réception d'informations électromagnétiques transmises par ledit dispositif médical courant.
- ladite interface homme-machine est adapté à la saisie d'informations relatives audit patient courant et pour déterminer ledit identifiant de patient courant à partir desdites informations.
- Le système comporte en outre des moyens pour déterminer un ensemble de dispositifs médicaux associés audit patient courant et pour afficher ledit ensemble sur une interface homme-machine.
- ladite interface homme-machine est également adaptée à la saisie d'informations relatives à un dispositif médical donné et pour déterminer un identifiant de dispositif médical donné à partir desdites informations, et dans lequel lesdits moyens de traitement sont prévus pour interroger ladite base de données afin de déterminer un ensemble d'identifiants de patients associés audit identifiant de dispositif médical donné.
- ladite base de données est en outre prévue pour associer un nombre de stock aux identifiants de dispositifs médicaux et pour décrémenter ledit nombre de stock lorsque l'identifiant de dispositif médical correspondant est déterminé comme identifiant de dispositif médical courant.
- Le système comprend en outre une interface réseau avec au moins un serveur d'achat de dispositifs médicaux, ladite interface réseau étant adapté pour transmettre audit serveur une commande d'achat lorsque le nombre de stock associé à un identifiant de dispositif médical devient inférieur à un seuil prédéterminé.
- Le système comprend en outre une interface d'étiquetage pour saisir des informations relatives à un dispositif médical donné et pour générer des informations visuelles destinées à être apposées sur ledit dispositif médical donné.

Un autre aspect de l'invention est relatif à un procédé pour la traçabilité des dispositifs médicaux utilisés pour le soin de patients, comportant
- une étape de détermination d'un identifiant de dispositif médical courant,
- une étape de détermination d'un identifiant de patient courant,
- une d'association dans une base de données d'identifiants de patients avec un ensemble d'identifiants de dispositifs médicaux utilisés, et
- étape d'insertion dudit identifiant de dispositif médical courant dans ladite base de données en association avec ledit identifiant de patient courant.

Suivant des modes de réalisation préférés, le procédé selon l'invention comprend une ou plusieurs des caractéristiques suivantes :
- Le procédé comporte une étape d'association dans ladite base de données d'un nombre de stock aux identifiants de dispositifs médicaux et de décrémentation dudit nombre de stock lorsque l'identifiant de dispositif médical correspondant est déterminé comme identifiant de dispositif médical courant.
- Le procédé comporte une étape de transmission à un serveur d'achat de dispositifs médicaux, d'une commande d'achat lorsque le nombre de stock associé à un identifiant de dispositif médical devient inférieur à un seuil prédéterminé.
- ledit identifiant de dispositif médical courant est déterminé par lecture d'informations visuelles apposées sur ledit dispositif médical.
- Le procédé comporte une étape de saisie de informations relatives à un dispositif médical donné afin de générer des informations visuelles destinées à être apposées sur ledit dispositif médical donné.

Un autre aspect de l'invention concerne un programme d'ordinateur comportant les moyens de code logiciel pour réaliser le procédé précédemment décrit lorsque mis en oeuvre sur une plateforme de traitement de l'information.

D'autres caractéristiques et avantages de l'invention apparaîtront à la lecture de la description qui suit d'un mode de réalisation préféré de l'invention, donnée à titre d'exemple et en référence au dessin annexé.

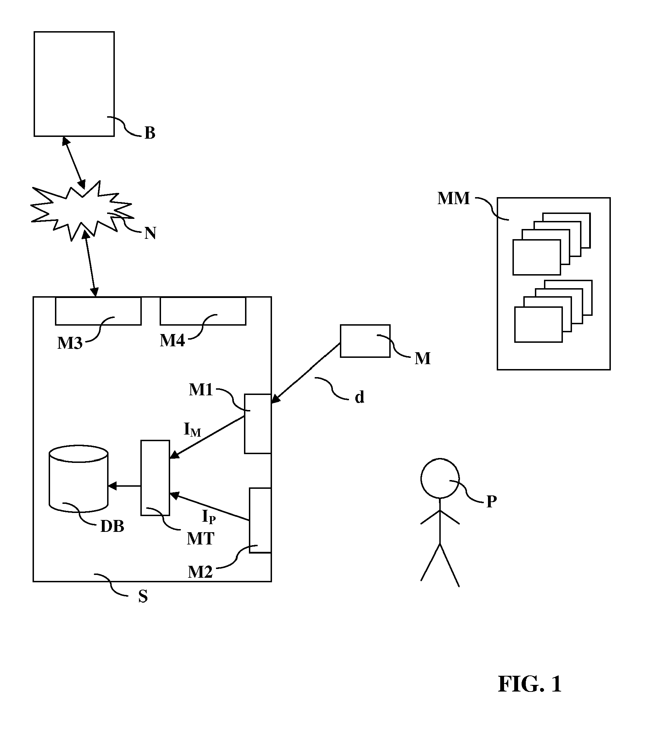

La figure 1 représente un système pour la traçabilité des dispositifs médicaux selon l'invention selon un mode de réalisation ainsi qu'un exemple de contexte dans lequel il peut s'inscrire.

Selon l'invention, un système S est installé au moins partiellement au sein de la structure de soins. La structure de soins peut être un hôpital, une clinique, un cabinet de médecine ou de dentisterie, ou tout autre établissement médical assujetti aux mêmes contraintes de traçabilité des dispositifs médicaux.

Ce dispositif comporte :
- une interface physique M1 pour déterminer automatiquement un identifiant I_{M} de dispositif médical courant M,
- une interface homme-machine M2 pour déterminer un identifiant Iₚ de patient courant P,
- une base de données DB associant des identifiants de patients avec un ensemble d'identifiants de dispositifs médicaux utilisés, et
- des moyens de traitement MT pour insérer l'identifiant I_{M} de dispositif médical courant M dans la base de données DB en association avec l'identifiant Iₚ de patient courant P.

Selon une mise en oeuvre de l'invention, le système peut être installé entièrement à proximité du site de soin, c'est-à-dire typiquement du lit ou du siège où s'assoit le patient P. Dans une telle mise en oeuvre, le système S peut être mis en oeuvre par une plateforme de traitement de l'information, telle un ordinateur, un tablette numérique, etc.

Selon d'autres mises en oeuvre de l'invention, le système S peut être réparti de sorte que certains seulement de ses moyens sont à proximité du site de soin, tandis que d'autres éléments peuvent être déportés. De telles mises en oeuvre peuvent être intéressantes dans le cadre d'une mutualisation du système S parmi en ensemble de sites de soins, dans le contexte d'un hôpital, d'un cabinet abritant plusieurs médecins ou dentistes, etc.

Notamment, la base de données DB peut être mutualisée, de même que les moyens de traitement MT au sein d'un serveur applicatif reliés par un réseau de communication à des terminaux ou postes de travail dans lesquels peuvent être implémentés les interfaces physiques M1 et les interfaces hommes-machines M2.

Selon une autre mise en oeuvre, la base de données et au moins une partie des moyens de traitement MT peuvent être déportés dans une autre localisation physique et connecté aux autres éléments du système S par un réseau de communication comme Internet. Une telle mise en oeuvre peut permettre la gestion sécurisée de la base de données par un prestataire de service spécialiste, ainsi que d'assurer la sécurité du contenu de la base de données en cas d'accident important impactant les infrastructures de la structure de soins.

Dans cette mise en oeuvre, la base de données peut être rendu accessible par une interface web depuis un poste de travail (un ordinateur, une tablette numérique, voire un terminal de communication de type Smartphone) localisé dans la structure de soin sur lequel est déployé un client web (par exemple un navigateur). La connexion entre le client web et le serveur web peut être sécurisée afin de garantir la confidentialité du contenu de la base de données DB.

La base de données peut en outre être une base hétérogène, constituée de plusieurs bases de données distinctes.

Par exemple, elle peut être constituée d'une base de données de patients, d'une base de données de dispositifs médicaux etc. Chacune de ces bases peut faire partie d'un produit logiciel distinct.

Les moyens de traitement MT permettent alors de faire le lien entre ces différentes bases et produits.

Il en résulte un avantage supplémentaire de l'invention de pouvoir s'assoir sur des produits existants et sur le contenu des bases de données déjà installées au sein de la structure de soins. Ainsi, l'invention ne nécessite pas de migrations coûteuses en temps et génératrices d'erreurs et permet au personnel de continuer à utiliser les outils dont il a l'habitude.

Lorsque des nouveaux dispositifs médicaux sont reçus par la structure de soins, ceux-ci peuvent être tout d'abord étiquetés.

Les dispositifs médicaux concernés peuvent comprendre ceux pour lesquels il existe une exigence légale de traçabilité, mais ils peuvent également comporter d'autres dispositifs en fonction de la politique propre souhaitée par le gérant de la structure de soins.

Pour ce faire, le système S peut disposer d'une interface d'étiquetage M4 qui permet de saisir des informations relatives à un dispositif médical donné. Ces informations peuvent notamment comprendre un nom de produit, un nom de fournisseur, un numéro de lot, une date de péremption, une date de livraison, etc.

Ces informations permettent de générer des informations visuelles destinées à être apposées sur le dispositif médical traité. Ces informations visuelles peuvent être une code barre ou bien un QR-code (code barre à deux dimensions) qui peuvent être imprimées sur des étiquettes autocollantes.

Les étiquettes peuvent ensuite être accolées sur les dispositifs médicaux traités et ces derniers peuvent ensuite être rangés dans un espace de rangement MM.

Les informations visuelles peuvent ne pas représenter directement l'ensemble des informations saisies mais uniquement une clé permettant d'identifier le dispositif médical de façon unique.

Le système S dispose en outre d'une base de données DB permettant d'associer cette clé unique aux informations saisies.

Aussi, la saisie des informations relatives à un dispositif médical donné peut permettre d'une part de générer une étiquette comportant des informations visuelles permettant de l'identifier, et d'autre part de mettre la base de données DB à jour afin de permettre de retrouver les informations saisies à partir des informations visuelles générées.

Cette étape d'étiquetage permet d'ensuite pouvoir mieux assurer la traçabilité de façon automatique et presque transparente pour le praticien lors d'un soin d'un patient P.

Lorsque celui-ci prend un dispositif médical de l'espace de rangement MM pour l'utiliser dans le cadre des soins à un patient P, il le présente à une interface physique M1 du système S.

Cette interface physique M1 est destinée à déterminer automatiquement un identifiant I_{M} du dispositif médical courant, c'est-à-dire qui lui est présenté.

Cet identifiant peut être la clé représentée par les informations visuelles imprimées sur l'étiquette apposée au dispositif médical courant M. L'interface physique M1 comporte pour ce faire un moyen de lecture d'informations visuelles d. Ce type de moyen peut être un scanner dédié à la lecture de code barre, ou QR codes, mais cela peut également être un moyen plus généraliste, comme un appareil photo numérique ou une caméra numérique, associé à un moyen logiciel de traitement d'images.

Selon un mode de réalisation, le système S peut posséder une interface physique comportant un moyen de réception d'informations électromagnétiques d transmises par le dispositif médical courant M.

Ce mode de réalisation peut faire usage des technologies de type « RFID » (pour Radio-Frequency Identification en langue anglaise). Cette technologie permet l'utilisation d'un champ électromagnétique pour transférer des informations à partir d'une étiquette (« *tag* ») électromagnétique apposée au dispositif médical M. L'étiquette peut ne pas nécessiter de source d'énergie propre et utiliser l'énergie du champ électromagnétique généré par le lecteur (intégré dans l'interface physique M1) pour transmettre les informations électromagnétiques.

Les deux modes de réalisations peuvent être implémentés simultanément au sein du système S, de sorte que des dispositifs médicaux étiquetés avec des codes-barres et dispositifs médicaux possédant des tags RFID peuvent être également traités.

Le système S dispose par ailleurs d'une interface homme-machine M2 prévue pour déterminer un identifiant Iₚ du patient courant P.

Cette interface homme-machine peut être adapté à la saisie d'informations relatives audit patient courant P et pour déterminer cet identifiant Iₚ de patient courant à partir des informations saisies.

Une telle interface-homme machine peut comprendre un clavier et/ou un écran tactile. Elle peut être également comprendre des moyens de reconnaissance vocale, de sorte que les informations peuvent être vocalisées et reconnues par le système S.

L'identifiant de patient peut être une clé identifiant chaque patient de la structure de soins de façon unique. Cela peut être un identifiant interne à la structure de soins, un identifiant interne au système S ou un identifiant public permettant d'identifier un patient de façon univoque à une échelle nationale. Cet identifiant public peut par exemple être le numéro d'identification de la sécurité sociale.

Les informations saisies peuvent comprendre notamment le nom, le prénom et le numéro d'identification de la sécurité sociale.

La fourniture de ces informations permet d'ouvrir le dossier virtuel associé au patient courant P au sein du système S.

Ce dossier virtuel peut être mémorisé dans la base de données DB.

Ainsi, lorsqu'un dispositif médical M est utilisé pour le patient, son identifiant I_{M} peut être automatiquement associé par les moyens de traitement MT à l'identifiant I_{P} de ce patient, et cette association peut être insérée au sein de la base de données.

Par exemple, la base de données DB peut contenir une table permettant de mettre en correspondance un identifiant I_{P} de patient avec un ensemble d'identifiants I_{M} de dispositifs médicaux. D'autres informations peuvent être également associés comme la date des soins ou le nom (ou l'identifiant) du personnel traitant.

Il est ainsi possible de déterminer aisément l'ensemble des dispositifs médicaux associés à un patient donné et de les afficher sur une interface homme machine (écran, sortie d'imprimante, etc.). Ceci peut être fait par les moyens de traitement MT en interrogeant la base de données DB.

En utilisant par exemple l'interface homme-machine M2, il est également possible de fournir des informations relatives à un dispositif médical donné et de déterminer un identifiant de ce dispositif médical donné à partir de ces informations.

Les moyens de traitement MT peuvent alors être prévus pour interroger la base de données DB afin de déterminer un ensemble d'identifiants de patients associés à cet identifiant de dispositif médical donné.

Il est alors possible d'afficher les identifiants de patients ainsi déterminés, par exemple sur un écran associé au système S ou bien d'en effectuer une sortie sur une imprimante, ou bien d'en exporter la liste vers une autre application logicielle.

Cette fonctionnalité permet notamment de répondre aux besoins de matériovigilance tels qu'exposés précédemment.

Ainsi, si un dispositif médical fait l'objet d'un rappel (à la suite d'un problème sanitaire détecté, d'un problème de fabrication, etc.), il est possible et aisé pour le gestionnaire de la structure de soins de déterminer les patients impactés. Il peut alors déclencher les actions appropriées.

Ces actions peuvent comprendre l'émission de courriers automatisée grâce à la mise en place d'une interface entre le système S et une application logicielle de messagerie. Ces actions peuvent également comprendre des actions manuelles comme des appels téléphoniques aux patients et la fixation de rendez-vous.

Par ailleurs, la base de données DB peut également être prévue pour associer un nombre de stock aux identifiants de dispositifs médicaux et pour décrémenter ce nombre de stock lorsque l'identifiant de dispositif médical correspondant M est déterminé comme identifiant de dispositif médical courant par l'interface physique M1.

Autrement dit, lorsqu'un dispositif médical M est utilisé pour le patient P, le nombre de stock associé est décrémenté d'une unité, de sorte que le nombre de stock contenu dans la base de données DB reflète en temps réel le nombre effectif de dispositif médical du même type dans l'espace de rangement MM.

Il est ainsi possible de déterminer à tout moment l'état des stocks pour chaque type de dispositif médical sans avoir à effectuer un inventaire au sein de l'espace de rangement MM.

Selon une mise en oeuvre de l'invention, il est en outre possible de fournir au système S une interface réseau M3 avec un ou plusieurs serveurs B d'achat de dispositifs médicaux. Ce ou ces serveurs B peuvent être accessibles à travers un réseau de télécommunication tel Internet.

Connaissant l'état des stocks, le système S peut utiliser cette interface réseau M3 pour transmettre au(x) serveur(s) B une commande d'achat lorsque le nombre de stock associé à un identifiant de dispositif médical devient inférieur à un seuil prédéterminé.

Ce seul prédéterminé peut dépendre du type de dispositif médical et/ou d'un délai moyen d'approvisionnement, etc.

Le nombre de stock initialement inséré dans la base de données DB pour chacun des types de dispositif médical peut être saisi lors d'une étape d'inventaire du contenu de l'espace de rangement MM. Il peut également être déterminé lors de l'étape d'étiquetage : lorsqu'un dispositif médical M est étiqueté, le nombre de stock associé à son type est incrémenté d'une unité (ou d'un nombre d'unités d'un lot).

D'autres interfaces peuvent être apportées au système S afin de le relier aux différentes applications logicielles métiers.

Il est par exemple possible, à l'issue d'une séance de soins, d'utiliser les informations associées dans la base de données DB aux dispositifs médicaux utilisés pour aider à générer l'ordonnance délivrée au patient P, ainsi que la facture (en prenant en compte le coût des dispositifs médicaux utilisés et facturable...).

Il est aussi possible de mutualiser les données stockées dans la base de données DB afin de fournir aux structures de soins un vaste panel de fonctionnalités au sein d'un système intégré et via des interfaces hommes-machines aussi uniformes que possibles. Ces fonctionnalités peuvent notamment comprendre : la gestion des patients et du cabinet (ou structure de soins), la gestion des stocks et de leur traçabilité, le télé-secrétariat et la gestion des agendas, la gestion des documents, la comptabilité, la gestion des prescriptions des patients, et la facturation.

Bien entendu, la présente invention n'est pas limitée aux exemples et au mode de réalisation décrits et représentés, mais elle est susceptible de nombreuses variantes accessibles à l'homme de l'art.

## Revendications

1. Système (S) pour la traçabilité des dispositifs médicaux utilisés pour le soin de patients, comportant une interface physique (M1) pour déterminer automatiquement un identifiant (I_{M}) de dispositif médical courant (M), une interface homme-machine (M2) pour déterminer un identifiant (Iₚ) de patient courant (P), une base de données (DB) associant des identifiants de patients avec un ensemble d'identifiants de dispositifs médicaux utilisés, et des moyens de traitement (MT) pour insérer ledit identifiant (I_{M}) de dispositif médical courant (M) dans ladite base de données (DB) en association avec ledit identifiant (Iₚ) de patient courant (P).

2. Système selon la revendication 1, dans lequel ladite interface physique (M1) comporte un moyen de lecture d'informations visuelles apposées sur ledit dispositif médical courant (M).

3. Système selon l'une des revendications 1, dans lequel ladite interface physique (M1) comporte un moyen de réception d'informations électromagnétiques transmises par ledit dispositif médical courant (M).

4. Système selon l'une des revendications précédentes, dans ladite interface homme-machine (M2) est adapté à la saisie d'informations relatives audit patient courant (P) et pour déterminer ledit identifiant (I_{P}) de patient courant à partir desdites informations.

5. Système selon l'une des revendications précédentes comportant en outre des moyens pour déterminer un ensemble de dispositifs médicaux associés audit patient courant et pour afficher ledit ensemble sur une interface homme-machine.

6. Système selon l'une des revendications précédentes, dans lequel ladite interface homme-machine (M2) est également adaptée à la saisie d'informations relatives à un dispositif médical donné et pour déterminer un identifiant de dispositif médical donné à partir desdites informations, et dans lequel lesdits moyens de traitement (MT) sont prévus pour interroger ladite base de données afin de déterminer un ensemble d'identifiants de patients associés audit identifiant de dispositif médical donné.

7. Système selon l'une des revendications précédentes dans lequel ladite base de données est en outre prévue pour associer un nombre de stock aux identifiants de dispositifs médicaux et pour décrémenter ledit nombre de stock lorsque l'identifiant de dispositif médical correspondant est déterminé comme identifiant de dispositif médical courant.

8. Système selon la revendication précédente comportant en outre une interface réseau (M3) avec au moins un serveur (B) d'achat de dispositifs médicaux, ladite interface réseau étant adapté pour transmettre audit serveur (B) une commande d'achat lorsque le nombre de stock associé à un identifiant de dispositif médical devient inférieur à un seuil prédéterminé.

9. Système selon l'une des revendications 2 à 8, comportant une interface d'étiquetage (M4) pour saisir des informations relatives à un dispositif médical donné et pour générer des informations visuelles destinées à être apposées sur ledit dispositif médical donné.

10. Procédé (S) pour la traçabilité des dispositifs médicaux utilisés pour le soin de patients, comportant
- une étape de détermination d'un identifiant (I_{M}) de dispositif médical courant (M),
- une étape de détermination d'un identifiant (Iₚ) de patient courant (P),
- une d'association dans une base de données d'identifiants de patients avec un ensemble d'identifiants de dispositifs médicaux utilisés, et
- étape d'insertion dudit identifiant (I_{M}) de dispositif médical courant (M) dans ladite base de données (DB) en association avec ledit identifiant (Iₚ) de patient courant (P).

11. Procédé selon la revendication précédant comportant une étape d'association dans ladite base de données (DB) d'un nombre de stock aux identifiants de dispositifs médicaux et de décrémentation dudit nombre de stock lorsque l'identifiant de dispositif médical correspondant est déterminé comme identifiant de dispositif médical courant.

12. Procédé selon la revendication précédente comportant une étape de transmission à un serveur (B) d'achat de dispositifs médicaux, d'une commande d'achat lorsque le nombre de stock associé à un identifiant de dispositif médical devient inférieur à un seuil prédéterminé.

13. Procédé selon l'une des revendications 10 à 12 dans lequel ledit identifiant (I_{M}) de dispositif médical courant est déterminé par lecture d'informations visuelles apposées sur ledit dispositif médical (M).

14. Procédé selon la revendication précédente comportant une étape de saisie de informations relatives à un dispositif médical donné afin de générer des informations visuelles destinées à être apposées sur ledit dispositif médical donné.

15. Programme d'ordinateur comportant les moyens de code logiciel pour réaliser le procédé selon l'une des revendications 10 à 14 lorsque mis en oeuvre sur une plateforme de traitement de l'information.
